# EUROPEAN PATENT APPLICATION

(11) **EP 4 574 834 A1**
(43) Date of publication of application: **25.06.2025**
(21) Application number: 23909008.7
(22) Date of filing: 07.06.2023
(51) Int. Cl.: C07K 1/10, C07K 5/04, C07K 7/04, C07C 303/40, C07C 311/04, C07C 231/02, C07C 233/47, C07C 269/06, C07C 271/22

(54) **METHOD FOR SYNTHESIZING AMIDE AND/OR POLYPEPTIDE WITH UNPROTECTED AMINO ACID AS AMINE COMPONENT**

(30) Priority: 28.12.2022 CN 202211696610
(71) Applicant: GUANGZHOU MEDICAL UNIVERSITY, Guangzhou, Guangdong 511436 (CN)
(72) Inventor: ZHAO, Junfeng, Guangzhou, Guangdong 511436 (CN); LIU, Tao, Guangzhou, Guangdong 511436 (CN); GUO, Xiao, Guangzhou, Guangdong 511436 (CN); PENG, Zejun, Guangzhou, Guangdong 511436 (CN)
(74) Representative: IP Sextant s.r.l.
(86) International application number: PCT/CN2023/098912
(87) International publication number: WO 2024/139061

(57) **Abstract**

The present invention provides a method for synthesizing an amide and/or a polypeptide using an unprotected amino acid as an ammonia component. In the method for preparing the polypeptide, an amino acid or peptide fragment with amino and carboxyl groups both unprotected is used as an ammonia component for the synthesis of a polypeptide, and after an α-acyloxyenamide derivative of a carboxylic acid forms an amide bond or peptide bond with the amino group of the unprotected amino acid or peptide fragment, the next peptide bond construction cycle can be carried out without removing the carboxyl protecting group.

## Description

### TECHNICAL FIELD

The present invention belongs to the technical field of organic synthetic chemistry, and in particular relates to a method for synthesizing an amide and/or a polypeptide using an unprotected amino acid as an ammonia component.

### BACKGROUND

Polypeptides and proteins are biological macromolecules with important functional activities, which are made up of natural amino acids linked together via amide bonds in a certain order and play an important role in regulating various life activities. A traditional method for constructing a peptide bond mainly involves converting a carboxylic acid into an active intermediate such as acyl chloride, an anhydride, an activated ester, or acyl azide by means of an activating agent or a condensing agent, and then reacting the active intermediate with another molecule of ammonia to form the peptide bond. Among various methods of forming peptide bonds, a condensing agent-based method is the most widely used method for polypeptide synthesis at present. Up to date, hundreds of condensing agents have been developed, and there are also more and more synthesis methods for polypeptides. However, the current methods of polypeptide synthesis are not ideal. Taking the synthesis of one dipeptide as an example: firstly, a carboxyl group of an *α*-amino acid and an amino group of another *α*-amino acid are necessarily temporarily protected. After the carboxyl group of the *α*-amino acid with the amino group protected forms a peptide bond with the amino group of the other *α*-amino acid with the carboxyl group protected, the amino or carboxyl protecting group is removed therefrom, and the next peptide bond is then constructed. By repeating the cycle of "protection", "condensation" and "deprotection" in this way, the target polypeptide can be obtained. The whole process of the polypeptide synthesis requires the consumption of a lot of protecting groups and deprotecting agents, which makes the atomic economy of the polypeptide synthesis process very low.

In 2016, some scholars designed and developed a new ynamide condensation agent for the synthesis of the amide bond and the peptide bond. This new condensing agent is easy to prepare, has good stability, small molecular weight, mild reaction conditions, and does not require any additives during use. More importantly, the α-chiral acid does not racemize in the condensing process, greatly improving the purity and yield of a product. However, this condensation agent still reacts with a carboxyl-protected amino acid as the ammonia component during the polypeptide synthesis. After the reaction is completed, the protected polypeptide is still obtained, and continuing to extend a peptide chain still requires a deprotection treatment, leading to a tedious polypeptide synthesis step, which is time-consuming and labor-consuming, and has a relatively high cost.

### SUMMARY

The present invention aims to solve the problem of low atom economy caused by the use of protecting groups in the above-mentioned existing polypeptide synthesis techniques. To this end, in a first aspect, the present invention provides a method for preparing an amide and/or a polypeptide, wherein an amino acid or peptide fragment with the amino and carboxyl groups both unprotected is used as an ammonia component for the synthesis of a polypeptide, and after an α-acyloxyenamide derivative of a carboxylic acid forms an amide bond or peptide bond with the amino group of the unprotected amino acid or peptide fragment, the next peptide bond construction cycle can be carried out without removing the carboxyl protecting group. On the other side, the ynamide condensing agent used in the present invention can effectively avoid racemization, that is, the condensing agent or the condensation method used in the related prior art for the synthesis of a polypeptide using unprotected amino acids can only result in the synthesis of a dipeptide, because serious racemization may be induced during the synthesis of a tripeptide or a polypeptide containing three or more amino acids. The method of the present invention avoids the racemization problem during polypeptide synthesis from unprotected amino acids, and the method can omit the steps of adding a protecting group to an amino acid and removing the protecting group from the product, so the use of chemicals and the generation of chemical wastes can be greatly reduced, making the polypeptide synthesis greener and more economical. This technical solution contributes to the "carbon peaking and carbon neutrality" economy by energy conservation and pollution reduction.

In a second aspect, the present invention provides another method for preparing an amide and/or a polypeptide.

In a third aspect, the present invention provides a use of the method for preparing an amide and/or a polypeptide.

According to the first aspect of the present invention, provided is a method for preparing an amide and/or a polypeptide, comprising the following steps: 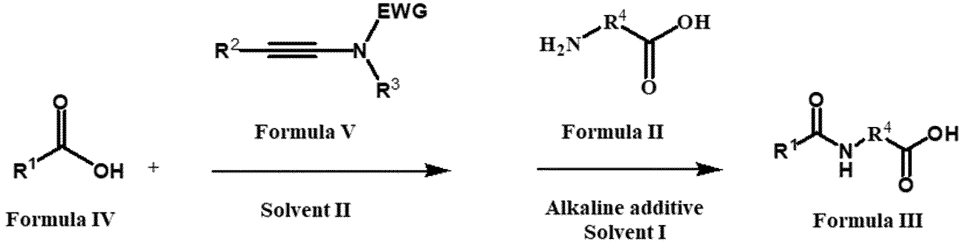
reacting a compound of Formula IV with a compound of Formula V in solvent II, and then carrying out a nucleophilic substitution reaction with a compound of Formula II and an alkaline additive in solvent I to prepare a compound of Formula III;
wherein R¹ is selected from alkyl, alkenyl, alkynyl, cycloalkyl, heterocycloalkyl, aryl, heteroaryl, an amino acid residual body lacking C-terminal carboxyl, an amino acid derivative residual body or a polypeptide fragment lacking C-terminal carboxyl; R² is selected from hydrogen, deuterium, alkyl, cycloalkyl, heterocycloalkyl, aryl, heteroaryl, silyl, or alkylsilyl; R³ is selected from alkyl, cycloalkyl, heterocycloalkyl, aryl, or heteroaryl; R⁴ is selected from alkyl, cycloalkyl, heterocycloalkyl, aryl, heteroaryl, an amino acid residue, or a polypeptide fragment; and EWG (electron-withdrawing group) is selected from nitro, cyano, sulfonyl, arylsulfonyl, alkanoyl, and phosphonyl; wherein the alkyl, alkenyl, alkynyl, cycloalkyl, heterocycloalkyl, aryl, heteroaryl, silyl, and alkylsilyl are optionally unsubstituted or substituted with one or more R¹¹, with each R¹¹ being independently selected from halogen, hydroxyl, carbonyl, and C₁-C₆ alkyl.

In some embodiments of the present invention, when the solvent II is different from the solvent I, the method for preparing the amide and/or polypeptide further comprises after the reaction, removing the solvent II before the nucleophilic substitution reaction.

In some preferred embodiments of the present invention, the aryl is selected from phenyl, o-tolyl, m-tolyl, p-tolyl, 2,4-xylyl, p-cumenyl, mesityl, 1-naphthyl, 2-naphthyl, 1-anthracenyl, 2-anthracenyl, 9-anthracenyl, 1-phenanthryl, 9-phenanthryl, 1-acenaphthenyl, 2-azulenyl, 1-pyrenyl, 2-triphenylene, o-biphenyl, m-biphenyl, p-biphenyl, and terphenyl.

In some more preferred embodiments of the present invention, the aromatic heterocyclic ring is selected from those having 1 or 2 nitrogen atoms, oxygen atoms or sulfur atoms, preferably as 5-10-membered heterocyclyl groups, for example, but not limited to triazolyl, 3-oxadiazolyl, 2-furanyl, 3-furanyl, 2-thienyl, 3-thienyl, 1-pyrrolyl, 2-pyrrolyl, 3-pyrrolyl, 2-pyridyl, 3-pyridyl, 4-pyridyl, 2-pyrazinyl, 2-oxazolyl, 3-isoxazolyl, 2-thiazolyl, 3-isothiazolyl, 2-imidazolyl, 3-pyrazolyl, 2-quinolyl, 3-quinolyl, 4-quinolyl, 5-quinolyl, 6-quinolyl, 7-quinolyl, 8-quinolyl, 1-isoquinolyl, 2-quinoxalinyl, 2-benzofuranyl, 2-benzothienyl, N-indolyl, and N-carbazolyl.

In some more preferred embodiments of the present invention, with regard to R¹, the amino acid residual body lacking C-terminal carboxyl or the amino acid derivative residual body lacking C-terminal carboxyl refers to the remaining part of an amino acid or amino acid derivative after a C-terminal carboxyl group was removed; preferably, the amino acid residual body lacking C-terminal carboxyl includes at least one of an α-amino acid residual body lacking C-terminal carboxyl, a β-amino acid residual body lacking C-terminal carboxyl, or a γ-amino acid residual body lacking C-terminal carboxyl; and preferably, the amino acid derivative residual body lacking C-terminal carboxyl includes an amino acid residual body lacking C-terminal carboxyl in which the amino group at C-terminal is protected, such as an amino acid residual body lacking C-terminal carboxyl in which the amino group at C-terminal is protected by N-alkoxycarbonyl and/or N-acyl. Preferably, the amino acid is generally represented by 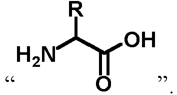 In this regard, as for R¹, the amino acid residual body lacking C-terminal carboxyl is 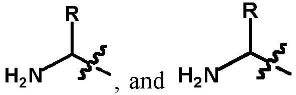 may be in R or S configuration. Specifically, the amino acid residual body lacking C-terminal carboxyl is selected from 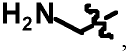 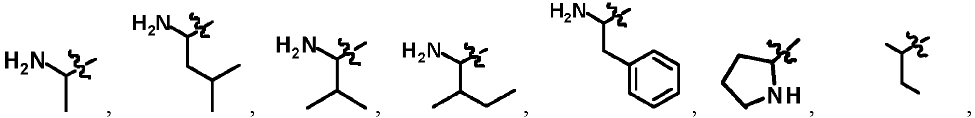 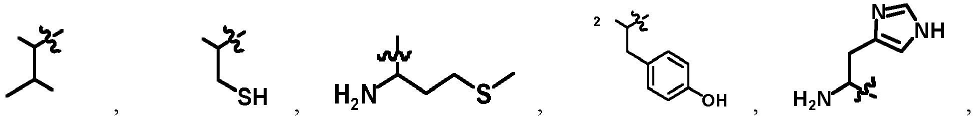 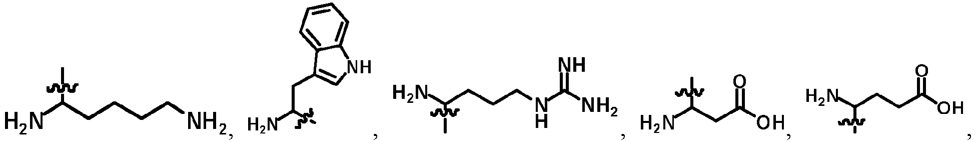 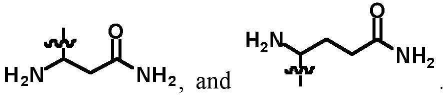 Specifically, the amino acid derivative residual body lacking C-terminal carboxyl is selected from an amino acid residual body lacking C-terminal carboxyl in which the amino group at C-terminal is protected by N-alkoxycarbonyl and/or N-acyl, among the above-mentioned amino acid residual body lacking C-terminal carboxyl.

According to the second aspect of the present invention, provided is another method for preparing an amide and/or a polypeptide, comprising the following steps: 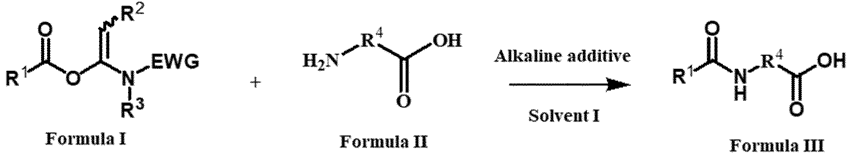
dissolving a compound of Formula I, a compound of Formula II and an alkaline additive in solvent I for a nucleophilic substitution reaction to prepare a compound of Formula III;
wherein R¹ is selected from alkyl, alkenyl, alkynyl, cycloalkyl, heterocycloalkyl, aryl, heteroaryl, an amino acid residual body lacking C-terminal carboxyl, an amino acid derivative residual body or a polypeptide fragment lacking C-terminal carboxyl; R² is selected from hydrogen, deuterium, alkyl, cycloalkyl, heterocycloalkyl, aryl, heteroaryl, silyl, or alkylsilyl; R³ is selected from alkyl, cycloalkyl, heterocycloalkyl, aryl, or heteroaryl; R⁴ is selected from alkyl, cycloalkyl, heterocycloalkyl, aryl, heteroaryl, an amino acid residue, or a polypeptide fragment; and EWG is selected from nitro, cyano, sulfonyl, arylsulfonyl, alkanoyl, and phosphonyl; wherein the alkyl, alkenyl, alkynyl, cycloalkyl, heterocycloalkyl, aryl, heteroaryl, silyl, and alkylsilyl are optionally unsubstituted or substituted with one or more R¹¹, with each R¹¹ being independently selected from halogen, hydroxyl, carbonyl, and C₁-C₆ alkyl.

In the present invention, the compound of Formula I is an α-acyloxyenamide compound, which is an activated ester in which the carbonyl group of the ester has good electrophilic activity and can undergo a nucleophilic substitution reaction with a nucleophile. An alkaline additive is used to free a non-nucleophilic or weakly nucleophilic ammonium ion in a compound of Formula II-A (a compound with both amino and carboxyl groups) into an amino group to form a compound of Formula I-A, so that the compound of Formula II-A and the compound of Formula I can undergo a nucleophilic substitution reaction without carboxyl protection to form an amide bond/peptide bond, so as to prepare an amide or polypeptide, i.e., a compound of Formula III, and at the same time form a byproduct, i.e., a compound of Formula IV. The reaction mechanism thereof is as follows:

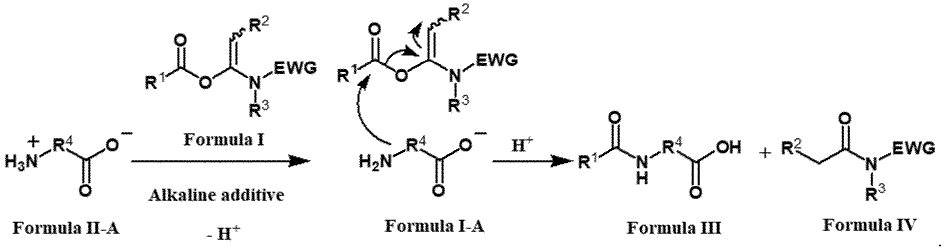

In some embodiments of the present invention, the alkaline additive includes an organic base and/or an inorganic base; preferably, the organic base includes at least one of trimethylamine, triethylamine, tripropylamine, N,N-dimethylethylamine, N,N-diethylmethylamine, N,N-diisopropylethylamine, N-methylpyrrolidine, N-methylpiperidine, N-methylmorpholine, tributylamine, tribenzylamine, dimethylbenzylamine, N,N-dimethylaniline, pyridine, 2-methylpyridine, 2,6-dimethylpyridine, 4-dimethylaminopyridine, quinoline, quinine, tetramethylguanidine, imidazole, 1,8-diazabicyclo[5.4.0]undec-7-ene, triethylene diamine, tetramethylethylenediamine, tetraethylethylenediamine, tetramethylpropylenediamine, tetraethylpropylenediamine, tetrabutylammonium iodide, tetrabutylammonium fluoride, tetrabutylammonium chloride, tetrabutylammonium bromide, or tetrabutylammonium hydroxide; and the inorganic base includes at least one of sodium bicarbonate, sodium carbonate, lithium bicarbonate, lithium carbonate, potassium bicarbonate, potassium carbonate, cesium bicarbonate, cesium carbonate, sodium hydroxide, potassium hydroxide, lithium hydroxide, cesium hydroxide, calcium bicarbonate, or calcium hydroxide.

In some preferred embodiments of the present invention, the molar ratio of the alkaline additive to the compound of Formula II is (0.5-20) : 1, preferably (1-15) : 1, further preferably (2-10) : 1.

In some more preferred embodiments of the present invention, the compound of Formula II is an organic compound with both amino and carboxyl groups; preferably, the compound of Formula II includes any one of an amino acid compound, a polypeptide compound with both free amino and carboxyl groups, or other compounds with both amino and carboxyl groups; further preferably, the compound of Formula II includes any one of an α-amino acid, a β-amino acid, a γ-amino acid, or a polypeptide compound in which neither the amino group nor the carboxyl group is protected; further more preferably, the compound of Formula II includes any one of glycine, alanine, leucine, isoleucine, proline, valine, phenylalanine, tyrosine, serine, threonine, cysteine, methionine, aspartic acid, glutamic acid, asparagine, glutamine, tryptophan, histidine, lysine, arginine, citrulline, or derivative compounds of the above amino acids. It should be understood that the twenty-one amino acids included for the compound of Formula II may all be natural α-amino acids in L-configuration and/or corresponding α-amino acids in D-configuration.

In some more preferred embodiments of the present invention, the solvent I includes at least one of water, acetonitrile, ethanol, isopropanol, tert-butanol, ethylene glycol, 1,2-propanediol, 2,3-butanediol, diethyl ether, ethylene glycol dimethyl ether, ethylene glycol diethyl ether, dioxane, dimethyl sulfoxide, N,N-dimethylformamide, N,N-dimethylacetamide, N-methylpyrrolidone, toluene, m-xylene, dichloromethane, 1,2-dichloroethane, or chloroform.

In some more preferred embodiments of the present invention, the temperature at which the nucleophilic substitution reaction is carried out is -20°C to 150°C, preferably 0°C to 90°C, further preferably 5°C to 50°C.

In some more preferred embodiments of the present invention, the method for preparing the amide and/or the polypeptide comprises the following steps: dissolving a compound of Formula II and an alkaline additive in solvent I, and then adding a compound of Formula I for a nucleophilic substitution reaction to prepare a compound of Formula III.

In some more preferred embodiments of the present invention, the method for preparing the amide and/or the polypeptide comprises the following steps: dissolving a compound of Formula II and an alkaline additive in solvent I under stirring for 0.5 min to 12 h, and then adding a compound of Formula I for a nucleophilic substitution reaction to prepare a compound of Formula III.

In some more preferred embodiments of the present invention, the method for preparing an amide and/or a polypeptide further comprises a step of subjecting the product resulting from the nucleophilic substitution reaction to purification, which comprises at least one of extraction, recrystallization, or column chromatography.

In some more preferred embodiments of the present invention, a preparation method for the compound of Formula I comprises the following steps: 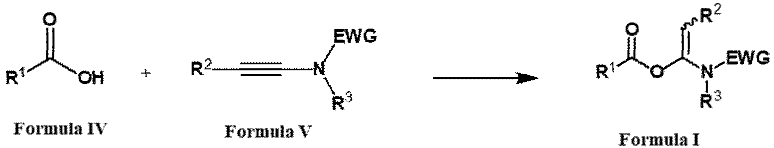
dissolving a compound of Formula IV and a compound of Formula V in solvent II to obtain a mixture, and reacting the mixture under stirring to prepare the compound of Formula I;
wherein the definitions of R¹, R², R³ and EWG are as defined previously.

In some more preferred embodiments of the present invention, the solvent II includes at least one of dichloromethane, chloroform, 1,2-dichloroethane, diethyl ether, toluene, acetonitrile, methanol, or ethanol.

In some more preferred embodiments of the present invention, the compound of Formula IV is a carboxylic acid compound; preferably, the compound of Formula IV includes at least one of a fatty acid, an aromatic acid, a heterocyclic acid, an acetylenic acid, an alkenoic acid, an N-acylcarbonylamino acid, an N-alkoxycarbonylamino acid, or a polypeptide carboxylic acid; further preferably, the compound of Formula IV includes at least one of N-benzyloxycarbonylamino acid, N-tert-butoxycarbonylamino acid, N-fluorenylmethoxycarbonylamino acid, N-acetylamino acid, or a polypeptide carboxylic acid.

In some more preferred embodiments of the present invention, the molar ratio of the compound of Formula IV to the compound of Formula V is 1 : (1-5), preferably 1 : (1.1-4), further preferably 1 : (1.2-3).

In some more preferred embodiments of the present invention, the temperature at which the stirred reaction is carried out is -20°C to 90°C, preferably 0°C to 50°C.

In some more preferred embodiments of the present invention, the method for preparing the amide and/or the polypeptide comprises the following steps: 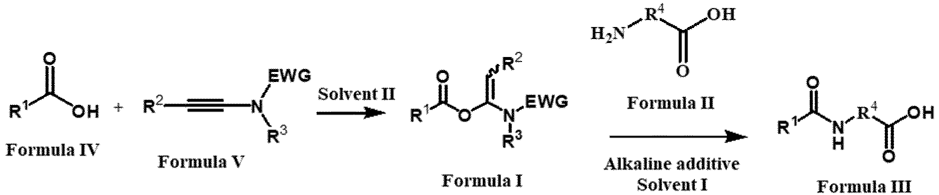
S1: dissolving a compound of Formula IV and a compound of Formula V in solvent II to obtain a mixture, and reacting the mixture under stirring to prepare a compound of Formula I; and
S2: dissolving the compound of Formula I, a compound of Formula II and an alkaline additive in solvent I for a nucleophilic substitution reaction to prepare a compound of Formula III;
wherein the definitions of R¹, R², R³, R⁴ and EWG are as defined previously.

According to a second aspect of the present invention, provided is a use of the method for preparing an amide and/or a polypeptide.

### Beneficial Effects of the Invention:

In the method for preparing an amide and/or a polypeptide according to the present invention, the use of low-cost unprotected amino acids as raw materials omits the step of adding a protecting group to the carboxyl group of an amino acid, and after the peptide bond is constructed, condensation directly with the amino group of the next amino acid can be carried out continuously without removing the protecting group. The method omits the use of group protecting agents, deprotecting agents, and solvents, thereby reducing the generation of substantial chemical wastes and also economizing human resources and energy consumption; furthermore, the cost of polypeptide synthesis is greatly reduced, the atomic economy and step economy of polypeptide synthesis are improved, energy conservation and emission reduction are efficiently achieved, and the efficiency of polypeptide synthesis is greatly improved, so that it has broad industrial application prospects.

### DETAILED DESCRIPTION

The concept and technical effects of the present invention will be described below clearly and completely in conjunction with examples, so as to fully understand the object, characteristics and effects of the present invention. Obviously, the described examples are merely some, rather than all, of the examples of the present invention. Based on the examples of the present invention, other examples obtained by those skilled in the art without involving any inventive effort all fall within the scope of protection of the present invention.

### EXAMPLE 1

In this example, a polypeptide of Formula 1 was prepared, and the specific process was as follows:

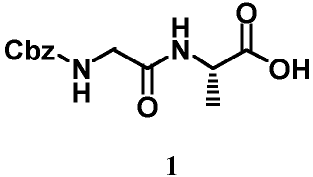

Cbz-Gly-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Ala-OH (1 mmol), triethylamine (1 mmol), 3 mL of N,N-dimethylformamide, and 1 mL of water were mixed under stirring in advance, and reacted. The reaction process was monitored by TLC. After the reaction was completed, 15 mL of water was added, the product was acidified to pH = 2 with 2 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with diethyl ether, and filtered to collect a solid, thus obtaining a pure product as a white solid with a yield of 95%.

The experimental data of magnetic resonance imaging and mass spectrometry of the product were as follows:
**¹H NMR** (400 MHz, DMSO) δ 12.63 (s, 1H), 8.15 (d, *J=* 7.2 Hz, 1H), 7.43 (t, *J=* 5.9 Hz, 1H), 7.38 - 7.30 (m, 5H), 5.04 (s, 2H), 4.29 - 4.19 (m, 1H), 3.71 - 3.62 (m, 2H), 1.28 (d, *J =* 7.2 Hz, 3H).
**¹³C NMR** (100 MHz, DMSO) δ = 174.1, 168.9, 156.5, 137.1, 128.4, 127.8, 127.7, 65.5, 47.5, 43.3, 17.4.
**HRMS (ESI)** m/z calcd. for C₁₃H₁₆N₂NaO₅ [M+Na]⁺: 303.0951, found: 303.0950.

### EXAMPLE 2

In this example, a polypeptide of Formula 2 was prepared, and the specific process was as follows:

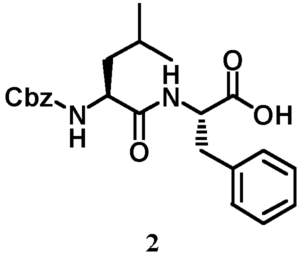

Cbz-Leu-OH (0.5 mmol) and N-methyl-N-methylsulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Phe-OH (1 mmol), N,N-diisopropylethylamine (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 2 with 2 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, and subjected to column chromatography isolation to obtain a pure product as a white solid, *dr >* 99 : 1, with a yield of 97%.

The experimental data of magnetic resonance imaging and mass spectrometry of the product were as follows:
**¹H NMR** (400 MHz, MeOD-*d*₄) δ 7.36 - 7.26 (m, 5H), 7.24 - 7.14 (m, 5H), 5.06 (s, 2H), 4.69 (dd, *J =* 8.0*,* 5.3 Hz, 1H), 4.26 - 4.14 (m, 1H), 3.18 (dd, *J =* 13.8, 5.1 Hz, 1H), 3.00 (dd, *J =* 13.8, 8.1 Hz, 1H), 1.67 - 1.59 (m, 1H), 1.52 - 1.41 (m, 2H), 0.96 - 0.85 (m, 6H).
**¹³C NMR** (100 MHz, MeOD-*d*₄) δ 174.9, 174.3, 158.2, 138.0, 138.0, 130.4, 129.4, 129.3, 128.9, 128.7, 127.7, 67.6, 54.7, 42.0, 38.3, 25.7, 23.4, 22.0.
**HRMS (ESI)** m/z calcd. for C₂₃H₂₈N₂NaO₅ [M+Na]⁺: 435.1890, found: 435.1883.

### EXAMPLE 3

In this example, a polypeptide of Formula 3 was prepared, and the specific process was as follows:

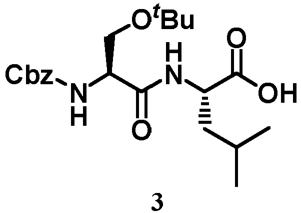

Cbz-Ser(tBu)-OH (0.5 mmol) and N-methyl-N-p-toluenesulfonyl ethynylamine (0.5 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Leu-OH (1 mmol), N,N-diisopropylethylamine (1 mmol), 3 mL of dimethylsulfoxide, and 1.5 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, and subjected to column chromatography isolation, and after filtration, a solid was collected to obtain a pure product as a white solid, *dr >* 99 : 1, with a yield of 93%.

The experimental data of magnetic resonance imaging and mass spectrometry of the product were as follows:
**¹H NMR** (400 MHz, DMSO-*d*₆) δ 7.98 (d, *J =* 7.9 Hz, 1H), 7.38 - 7.28 (m, 5H), 7.21 (d, *J =* 8.5 Hz, 1H), 5.04 (s, 2H), 4.29 - 4.23 (m, 1H), 4.18 - 4.12 (m, 1H), 3.50 - 3.43 (m, 2H), 1.70 - 1.62 (m, 1H), 1.56 - 1.47 (m, 2H), 1.10 (s, 9H), 0.86 (dd, *J =* 16.6, 6.3 Hz, 6H).
**¹³C NMR** (100 MHz, DMSO-*d*₆) δ 173.9, 169.7, 155.8, 137.0, 128.3, 127.8, 127.7, 72.8, 65.5, 62.0, 55.4, 50.3, 40.3, 27.2, 24.1, 22.9, 21.4.
**HRMS (ESI)** m/z calcd. for C₂₁H₃₂N₂NaO₆ [M+Na]⁺: 431.2153, found: 431.2149.

### EXAMPLE 4

In this example, a polypeptide of Formula 4 was prepared, and the specific process was as follows:

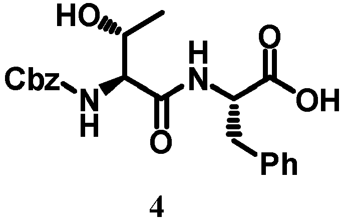

Cbz-Thr-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Leu-OH (1 mmol), sodium carbonate (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 2 with 2 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with diethyl ether, and filtered to collect a solid, thus obtaining a pure product as a white solid, *dr >* 99 : 1, with a yield of 94%.

The experimental data of magnetic resonance imaging and mass spectrometry of the product were as follows:
**¹H NMR** (400 MHz, DMSO-*d*₆) δ 8.06 (d, *J =* 7.7 Hz, 1H), 7.41 - 7.17 (m, 10H), 6.95 (d, *J* = 8.7 Hz, 1H), 5.05 (s, 2H), 4.48 (q, *J =* 7.3 Hz, 1H), 3.96 (dd, *J =* 8.2, 5.2 Hz, 1H), 3.88 - 3.76 (m, 1H), 3.05 (dd, *J =* 13.7, 5.0 Hz, 1H), 2.93 (dd, *J =* 13.6, 8.1 Hz, 1H), 1.01 (d, *J =* 6.1 Hz, 3H).
**¹³C NMR** (100 MHz, DMSO-*d*₆) δ 172.6, 170.0, 156.0, 137.3, 137.0, 129.2, 128.3, 128.1, 127.8, 127.7, 126.4, 66.8, 65.5, 60.5, 53.3, 36.9, 19.6.
**HRMS (ESI)** m/z calcd. for C₂₁H₂₄N₂NaO₆ [M+Na]⁺: 423.1527, found: 423.1524.

### EXAMPLE 5

In this example, a polypeptide of Formula 5 was prepared, and the specific process was as follows:

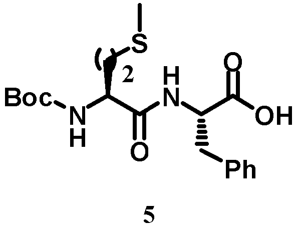

Boc-Met-OH (0.5 mmol) and N-methyl-N-p-toluenesulfonyl ethynylamine (0.5 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Leu-OH (1 mmol), triethylamine (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, and subjected to column chromatography isolation to obtain a pure product as a white solid, *dr >* 99 : 1, with a yield of 94%.

The experimental data of magnetic resonance imaging and mass spectrometry of the product were as follows:
**¹H NMR** (400 MHz, DMSO-*d*₆) δ 7.97 (d, *J =* 7.8 Hz, 1H), 7.34 - 7.16 (m, 5H), 6.91 (d, *J =* 8.2 Hz, 1H), 4.46 (q, *J =* 8.0 Hz, 1H), 4.01 (q, *J =* 7.9 Hz, 1H), 3.07 (dd, *J =* 13.8, 4.9 Hz, 1H), 2.92 (dd, *J =* 13.9, 8.7 Hz, 1H), 2.45 - 2.29 (m, 2H), 2.01 (s, 3H), 1.85 - 1.64 (m, 2H), 1.37 (s, 9H).
**¹³C NMR** (100 MHz, DMSO-*d*₆) δ 172.8, 171.6, 155.2, 137.3, 129.2, 128.1, 126.4, 78.2, 53.6, 53.2, 36.7, 31.9, 29.6, 28.2, 14.6.
**HRMS (ESI)** m/z calcd. for C₁₉H₂₈N₂NaO₅S [M+Na]⁺: 419.1611, found: 419.1609.

### EXAMPLE 6

In this example, a polypeptide of Formula 6 was prepared, and the specific process was as follows:

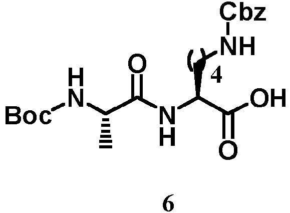

Boc-Ala-OH (0.5 mmol) and N-methyl-N-methylsulfonyl ethynylamine (0.5 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Lys(Cbz)-OH (1 mmol), potassium carbonate (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, and subjected to column chromatography isolation to obtain a pure product as a white solid, *dr >* 99 : 1, with a yield of 94%.

The experimental data of magnetic resonance imaging and mass spectrometry of the product were as follows:
**¹H NMR** (400 MHz, DMSO-*d*₆) δ 7.88 (d, *J =* 7.8 Hz, 1H), 7.39 - 7.27 (m, 5H), 7.20 (t, *J =* 5.8 Hz, 1H), 6.86 (d, *J =* 7.7 Hz, 1H), 5.00 (s, 2H), 4.20 - 4.13 (m, 1H), 4.03 - 3.96 (m, 1H), 3.01 - 2.94 (m, 2H), 1.75 - 1.65 (m, 1H), 1.63 - 1.53 (m, 1H), 1.37 (s, 9H), 1.34 - 1.26 (m, 3H), 1.17 (d, *J* = 7.1 Hz, 3H).
**¹³C NMR** (100 MHz, DMSO-*d*₆) δ 173.6, 172.8, 156.1, 155.1, 137.3, 128.4, 127.8, 127.7, 78.1, 65.2, 51.7, 49.6, 40.1, 30.9, 29.0, 28.2, 22.6, 18.1.
**HRMS (ESI)** m/z calcd. for C₂₂H₃₄N₂O₇ [M+H]⁺: 452.2391, found: 452.2390.

### EXAMPLE 7

In this example, a polypeptide of Formula 7 was prepared, and the specific process was as follows:

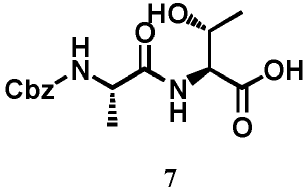

Cbz-Ala-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Thr-OH (1 mmol), sodium bicarbonate (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 2 with 2 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with ethyl acetate and diethyl ether, and filtered to collect a solid, thus obtaining a pure product as a white solid, *dr >* 99 : 1, with a yield of 92%.

The experimental data of magnetic resonance imaging and mass spectrometry of the product were as follows:
**¹H NMR** (400 MHz, DMSO-*d*₆) δ 7.67 (d, *J =* 8.6 Hz, 1H), 7.53 (d, *J =* 7.7 Hz, 1H), 7.39 - 7.26 (m, 5H), 5.03 (s, 2H), 4.25 - 4.14 (m, 3H), 1.24 (d, *J =* 7.1 Hz, 3H), 1.06 (d, *J =* 6.1 Hz, 3H).
**¹³C NMR** (100 MHz, DMSO-*d*₆) δ 173.0, 172.2, 155.8, 137.1, 128.5, 127.9, 127.8, 66.5, 65.5, 57.5, 50.2, 20.4, 18.2.
**HRMS (ESI)** m/z calcd. for C₁₅H₂₀N₂O₆ [M+Na]⁺: 347.1214, found: 347.1221.

### EXAMPLE 8

In this example, a polypeptide of Formula 8 was prepared, and the specific process was as follows:

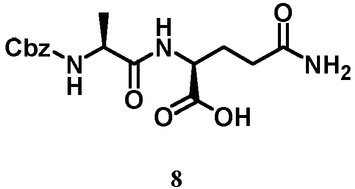

Cbz-Ala-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, H-Gln-OH (1 mmol) was added, 3 mL of acetonitrile and 1.5 mL of water were added as a solvent, and N,N-diisopropylethylamine (1 mmol) was added. The reaction process was monitored by TLC. After the reaction was completed, 15 mL of water was added, the product was acidified to pH = 1 with 2 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 97%.

The experimental data of magnetic resonance imaging and mass spectrometry of the product were as follows:
**¹H NMR** (400 MHz, DMSO-*d*₆) δ 8.13 (d, *J =* 7.6 Hz, 1H), 7.59 - 7.12 (m, 7H), 6.79 (s, 1H), 5.09 - 4.95 (m, 2H), 4.22 - 4.05 (m, 2H), 2.22 - 2.07 (m, 2H), 2.01 - 1.91 (m, 1H), 1.86 - 1.73 (m, 1H), 1.22 (d, *J =* 7.1 Hz, 3H).
**¹³C NMR** (100 MHz, DMSO-*d*₆) δ 173.6, 173.3, 172.7, 155.7, 137.1, 128.4, 127.8, 127.8, 65.4, 51.6, 49.9, 31.4, 27.0, 18.2.
**HRMS (ESI)** m/z calcd. for C₁₆H₂₁N₃NaO₆ [M+Na]⁺: 374.1323, found: 374.1321.

### EXAMPLE 9

In this example, a polypeptide of Formula 9 was prepared, and the specific process was as follows:

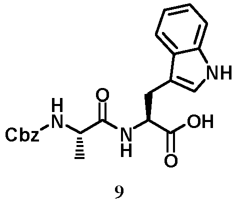

Cbz-Ala-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, dichloromethane was removed by distillation under reduced pressure, and the product was transferred to a system, in which H-Trp-OH (1 mmol), cesium carbonate (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 1 with 2 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, and subjected to column chromatography isolation to obtain a pure product as a light yellow solid, *dr >* 99 : 1, with a yield of 93%.

The experimental data of magnetic resonance imaging and mass spectrometry of the product were as follows:
**¹H NMR** (400 MHz, DMSO-*d*₆) δ 10.89 (s, 1H), 8.12 - 8.03 (m, 1H), 7.57 (t, *J =* 6.0 Hz, 1H), 7.44 (dd, *J =* 8.1, 4.1 Hz, 1H), 7.34 (d, *J =* 15.7 Hz, 8H), 7.20 (s, 1H), 7.07 (s, 1H), 6.98 (s, 1H), 5.13 - 4.94 (m, 3H), 4.54 (s, 1H), 4.15 (q, *J =* 5.8, 4.9 Hz, 2H), 3.22 (d, *J =* 14.3 Hz, 2H), 3.17 - 3.05 (m, 2H), 1.26 - 1.19 (m, 7H).
**¹³C NMR** (100 MHz, DMSO-*d*₆) δ 173.3, 172.6, 155.7, 137.1, 136.1, 128.4, 127.8, 127.8, 127.4, 123.8, 121.0, 118.4, 118.3, 111.4, 109.7, 65.5, 53.0, 50.0, 27.1, 18.3.
**HRMS (ESI)** m/z calcd. for C₂₂H₂₃N₃NaO₅ [M+Na]⁺: 432.1530, found: 432.1530.

### EXAMPLE 10

In this example, a polypeptide of Formula 10 was prepared, and the specific process was as follows:

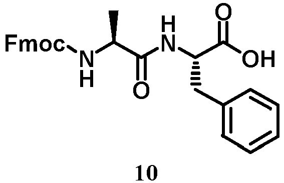

Fmoc-Ala-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Phe-OH (1 mmol), N,N-diisopropylethylamine (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 1 with 2 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, and subjected to column chromatography isolation to obtain a pure product as a white solid, *dr >* 99 : 1, with a yield of 91%.

The experimental data of magnetic resonance imaging and mass spectrometry of the product were as follows:
**¹H NMR** (400 MHz, DMSO-*d*₆) δ 8.02 (d, *J =* 7.5 Hz, 1H), 7.89 (d, *J =* 7.5 Hz, 2H), 7.74 (d, *J* = 6.8 Hz, 1H), 7.48 (d, *J =* 7.7 Hz, 1H), 7.42 (t, *J =* 7.4 Hz, 2H), 7.33 (t, *J =* 7.3 Hz, 2H), 7.27 - 7.16 (m, 4H), 4.50 - 4.39 (m, 1H), 4.26 (d, *J =* 6.1 Hz, 1H), 4.24 - 4.18 (m, 1H), 4.12 - 4.06 (m, 1H), 3.07 (dd, *J =* 13.7, 4.8 Hz, 1H), 2.93 (dd, *J =* 13.7, 8.4 Hz, 1H), 1.20 (d, *J =* 7.1 Hz, 3H).
**¹³C NMR** (100 MHz, DMSO-*d*₆) δ 172.8, 172.4, 155.6, 143.9, 143.8, 140.7, 137.4, 129.2, 128.1, 127.6, 127.1, 126.3, 125.3, 120.1, 65.7, 53.5, 49.9, 46.6, 36.7, 18.2.
**HRMS (ESI)** m/z calcd. for C₂₂H₂₃N₃NaO₅ [M+Na]⁺: 432.1530, found: 432.1530.

### EXAMPLE 11

In this example, a polypeptide of Formula 11 was prepared, and the specific process was as follows:

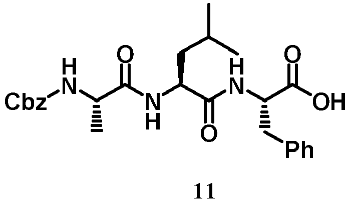

Cbz-Ala-Leu-OH (0.5 mmol) and N-methyl-N-p-toluenesulfonyl ethynylamine (0.5 mmol) were taken and dissolved in 5 mL of dichloromethane, and the mixture was reacted under stirring at room temperature. The reaction process was monitored by TLC. When the raw material acid was completely consumed, the product was transferred to a system, in which H-Phe-OH (1 mmol), N,N-diisopropylethylamine (1 mmol), and 5 mL of ethylene glycol were mixed under stirring in advance, and reacted. The reaction process was monitored by TLC. After the reaction was completed, 15 mL of water was added, the product was acidified to pH = 1 with 2 M hydrochloric acid, the aqueous phase was extracted twice with dichloromethane, the organic phases were combined, washed once with water and once with a saturated table salt solution, dried over anhydrous sodium sulfate, distilled under reduced pressure to remove the solvent, recrystallized with diethyl ether, and filtered to collect a solid, thus obtaining a pure product as a white solid, *dr >* 99 : 1, with a yield of 93%.

The experimental data of magnetic resonance imaging and mass spectrometry of the product were as follows:
**¹H NMR** (400 MHz, DMSO-*d*₆) δ 8.04 (d, *J =* 7.6 Hz, 1H), 7.86 (d, *J =* 8.3 Hz, 1H), 7.43 (d, *J* = 7.6 Hz, 1H), 7.37 - 7.17 (m, 10H), 5.02 (s, 2H), 4.47 - 4.41 (m, 1H), 4.36 - 4.30 (m, 1H), 4.10 - 4.04 (m, 1H), 3.07 (dd, *J =* 13.9, 5.2 Hz, 1H), 2.93 (dd, *J =* 13.8, 8.5 Hz, 1H), 1.67 - 1.50 (m, 1H), 1.43 (d, *J =* 6.7 Hz, 1H), 1.17 (d, *J =* 7.0 Hz, 3H), 0.90 - 0.76 (m, 6H).
**¹³C NMR** (100 MHz, DMSO-*d*₆) δ 172.8, 172.2, 171.9, 155.7, 137.5, 137.1, 129.2, 128.4, 128.2, 127.8, 127.8, 126.4, 65.4, 53.4, 51.0, 50.1, 41.1, 36.7, 24.1, 23.1, 21.8, 18.2.
**HRMS (ESI)** m/z calcd. for C₂₆H₃₃N₃NaO₆ [M+Na]⁺: 506.2262, found: 506.2253.

### EXAMPLE 12

In this example, a polypeptide of Formula 12 was prepared, and the specific process was as follows:

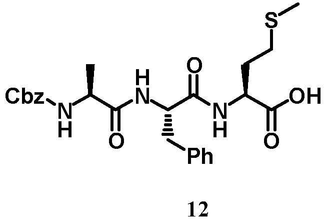

Cbz-Ala-Phe-OH (0.5 mmol) and N-methyl-N-p-toluenesulfonyl ethynylamine (0.5 mmol) were taken and dissolved in 5 mL of dichloromethane, and the mixture was reacted under stirring at room temperature. The reaction process was monitored by TLC. When the raw material acid was completely consumed, the product was transferred to a system, in which H-Met-OH (1 mmol), N,N-diisopropylethylamine (1 mmol), and 5 mL of 2,3-butanediol were mixed under stirring in advance, and reacted. The reaction process was monitored by TLC. After the reaction was completed, 15 mL of water was added, the product was acidified to pH = 1 with 2 M hydrochloric acid, the aqueous phase was extracted twice with dichloromethane, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 93%.

The experimental data of magnetic resonance imaging and mass spectrometry of the product were as follows:
**¹H NMR** (400 MHz, DMSO-*d*₆) δ 8.18 (d, *J =* 7.3 Hz, 1H), 7.94 (d, *J =* 7.7 Hz, 1H), 7.43 (d, *J* = 7.0 Hz, 1H), 7.40 - 7.12 (m, 10H), 5.10 - 4.93 (m, 2H), 4.60 - 4.51 (m, 1H), 4.41 - 4.34 (m, 1H), 4.05 - 3.98 (m, 1H), 3.14 - 3.03 (m, 1H), 2.91 - 2.79 (m, 1H), 2.52 - 2.42 (m, 2H), 2.04 (s, 3H), 2.02 - 1.95 (m, 1H), 1.93 - 1.85 (m, 1H), 1.13 (d, *J =* 7.1 Hz, 3H).
**¹³C NMR** (100 MHz, DMSO-*d*₆) δ 173.2, 172.3, 171.0, 155.8, 137.7, 137.0, 129.4, 128.4, 128.0, 127.9, 127.8, 126.3, 65.6, 53.6, 51.1, 50.4, 37.3, 30.9, 29.7, 18.1, 14.7.
**HRMS (ESI)** m/z calcd. forC₂₅H₃₁N₃NaO₆S [M+Na]⁺: 524.1826, found: 524.1824.

### EXAMPLE 13

In this example, a polypeptide of Formula 13 was prepared, and the specific process was as follows:

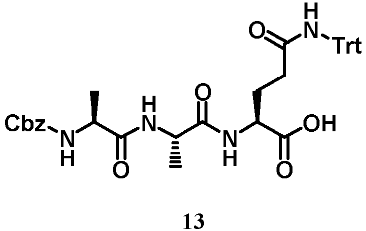

Cbz-Ala-Ala-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Gln(Trt)-OH (1 mmol), sodium bicarbonate (2 mmol), and 5 mL of dichloromethane were mixed under stirring in advance, and reacted. The reaction process was monitored by TLC. After the reaction was completed, 15 mL of water was added, the product was acidified to pH = 3 with 10% citric acid, the aqueous phase was extracted once with ethyl acetate and washed twice with water, the organic phase was washed once with a saturated table salt solution, dried over anhydrous sodium sulfate, distilled under reduced pressure to remove the solvent, recrystallized with dichloromethane and diethyl ether, and filtered to collect a solid, thus obtaining a pure product as a light yellow solid, *dr >* 99 : 1, with a yield of 90%.

The experimental data of magnetic resonance imaging and mass spectrometry of the product were as follows:
**¹H NMR** (400 MHz, DMSO-*d*₆) δ 8.59 (s, 1H), 8.05 (d, *J =* 8.2 Hz, 1H), 7.93 (t, *J =* 6.5 Hz, 1H), 7.44 (t, *J =* 5.9 Hz, 1H), 7.38 - 7.14 (m, 22H), 5.06 - 4.97 (m, 2H), 4.37 - 4.29 (m, 1H), 4.22 - 4.15 (m, 1H), 4.11 - 4.05 (m, 1H), 2.41 - 2.29 (m, 2H), 1.99 - 1.88 (m, 1H), 1.79 - 1.69 (m, 1H), 1.26 - 1.16 (m, 6H).
**¹³C NMR** (100 MHz, DMSO-*d*₆) δ 173.3, 172.1, 172.0, 171.2, 155.7, 144.9, 137.0, 128.5, 128.3, 127.8, 127.7, 127.4, 126.3, 69.3, 65.4, 51.6, 50.0, 47.9, 32.6, 27.3, 18.3, 18.1.
**HRMS (ESI)** m/z calcd. for C₃₈H₄₀N₄NaO₇ [M+Na]⁺: 687.2789, found: 687.2874.

### EXAMPLE 14

In this example, a polypeptide of Formula 14 was prepared, and the specific process was as follows:

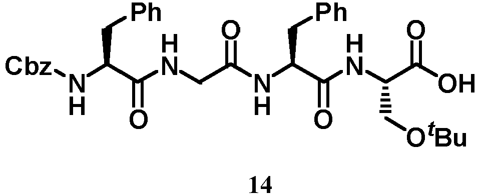

Cbz-Phe-Gly-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Phe-Ser(tBu)-OH (1 mmol), N,N-diisopropylethylamine (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 92%.

The experimental data of magnetic resonance imaging and mass spectrometry of the product were as follows:
**¹H NMR** (400 MHz, DMSO-*d*₆) δ 8.33 - 8.17 (m, 2H), 8.05 (d, *J=* 8.4 Hz, 1H), 7.52 (d, *J =* 8.5 Hz, 1H), 7.38 - 7.13 (m, 15H), 5.02 - 4.87 (m, 2H), 4.77 - 4.65 (m, 1H), 4.43 - 4.35 (m, 1H), 4.32 - 4.25 (m, 1H), 3.80 - 3.74 (m, 1H), 3.69 - 3.60 (m, 2H), 3.57 - 3.52 (m, 1H), 3.11 - 2.98 (m, 2H), 2.83 - 2.68 (m, 2H), 1.13 (s, 9H).
**¹³C NMR** (100 MHz, DMSO-*d*₆) δ 171.8, 171.6, 171.1, 168.4, 155.9, 138.2, 137.8, 137.0, 129.3, 129.2, 128.3, 128.2, 128.1, 128.0, 127.7, 127.4, 126.3, 72.9, 65.3, 61.6, 56.2, 53.6, 53.1, 41.9, 37.8, 37.5, 27.2.
**HRMS (ESI)** m/z calcd. for C₃₅H₄₂N₄NaO₈ [M+Na]⁺: 669.2895, found: 669.2900.

### EXAMPLE 15

In this example, a polypeptide of Formula 15 was prepared, and the specific process was as follows:

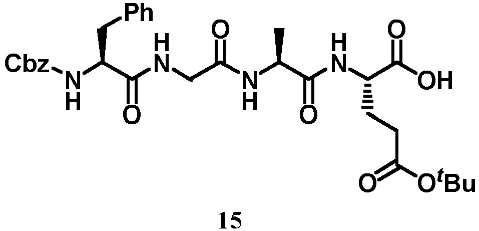

Cbz-Phe-Gly-Ala-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Glu(tBu)-OH (1 mmol), N,N-diisopropylethylamine (1 mmol), and 5 mL of 2,3-butanediol were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 89%.

The experimental data of magnetic resonance imaging and mass spectrometry of the product were as follows:
**¹H NMR** (400 MHz, DMSO-*d*₆) δ 8.38 - 8.28 (m, 1H), 8.14 (d, *J =* 6.9 Hz, 1H), 7.96 (d, *J =* 6.0 Hz, 1H), 7.57 (d, *J =* 7.6 Hz, 1H), 7.35 - 7.15 (m, 10H), 4.95 (s, 2H), 4.41 - 4.18 (m, 3H), 3.82 - 3.71 (m, 2H), 3.10 - 3.01 (m, 1H), 2.83 - 2.71 (m, 1H), 2.36 - 2.18 (m, 2H), 2.05 - 1.90 (m, 1H), 1.88 - 1.72 (m, 1H), 1.38 (s, 9H), 1.27 - 1.19 (m, 3H).
**¹³C NMR** (100 MHz, DMSO-*d*₆) δ 173.1, 172.2, 171.9, 171.5, 168.2, 156.0, 138.1, 137.0, 129.2, 128.3, 128.0, 127.7, 127.4, 126.2, 79.8, 65.3, 56.3, 51.1, 47.9, 42.1, 37.3, 31.3, 27.7, 26.4, 18.3.
**HRMS (ESI)** m/z calcd. for C₃₁H₄₁N₄O₉ [M+H]⁺: 613.2868, found: 613.2865.

### EXAMPLE 16

In this example, a polypeptide of Formula 16 was prepared, and the specific process was as follows:

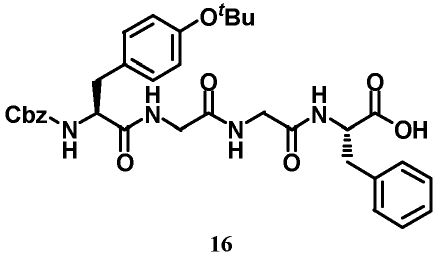

Cbz-Tyr(tBu)-Gly-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the dichloromethane was removed by distillation under reduced pressure, and the product was transferred to a system, in which H-Gly-Phe-OH (1 mmol), sodium carbonate (1 mmol), 3 mL of acetonitrile, and 1.5 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 96%.

The experimental data of magnetic resonance imaging and mass spectrometry of the product were as follows:
**¹H NMR** (400 MHz, DMSO-*d*₆) δ 8.38 - 8.26 (m, 1H), 8.18 (d, *J =* 6.6 Hz, 1H), 8.11 - 7.96 (m, 1H), 7.64 - 7.43 (m, 1H), 7.36 - 7.15 (m, 12H), 6.86 (d, *J =* 7.4 Hz, 2H), 5.01 - 4.86 (m, 2H), 4.53 - 4.43 (m, 1H), 4.35 - 4.26 (m, 1H), 3.84 - 3.70 (m, 4H), 3.12 - 3.00 (m, 2H), 2.97 - 2.88 (m, 1H), 2.80 - 2.69 (m, 1H), 1.26 (s, 9H).
**¹³C NMR** (100 MHz, DMSO-*d*₆) δ 172.8, 172.0, 169.0, 168.6, 156.0, 153.5, 137.5, 137.0, 132.8, 129.8, 129.2, 128.3, 128.3, 127.7, 127.5, 126.5, 123.3, 77.6, 65.4, 56.3, 53.6, 42.2, 41.7, 36.9, 36.8, 28.6.

### EXAMPLE 17

In this example, a polypeptide of Formula 17 was prepared, and the specific process was as follows:

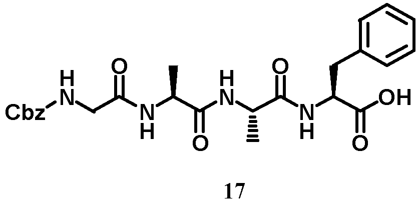

Cbz-Gly-Ala-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Ala-Phe-OH (1 mmol), N,N-diisopropylethylamine (1 mmol), and 5 mL of 2,3-butanediol were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 89%.

The experimental data of magnetic resonance imaging and mass spectrometry of the product were as follows:
**¹H NMR** (400 MHz, DMSO-*d*₆) δ 8.00 (t, *J =* 6.8 Hz, 3H), 7.51 - 7.41 (m, 1H), 7.42 - 7.16 (m, 10H), 5.04 (s, 2H), 4.47 - 4.38 (m, 1H), 4.31 (p, *J =* 7.2 Hz, 2H), 3.66 (d, *J =* 5.9 Hz, 2H), 3.06 (dd, *J =* 13.8, 5.1 Hz, 1H), 2.93 (dd, *J =* 13.6, 8.5 Hz, 1H), 1.19 (t, *J =* 8.1 Hz, 6H).
**¹³C NMR** (100 MHz, DMSO-*d*₆) δ 172.7, 172.0, 171.7, 168.8, 156.5, 137.4, 137.1, 129.2, 128.4, 128.2, 127.8, 127.7, 126.5, 65.5, 53.5, 48.0, 43.5, 36.7, 18.4, 18.1.
**HRMS (ESI)** m/z calcd. for C₂₅H₃₀N₄NaO₇ [M+Na]⁺: 521.2007, found: 521.2009.

### EXAMPLE 18

In this example, a polypeptide of Formula 18 was prepared, and the specific process was as follows:

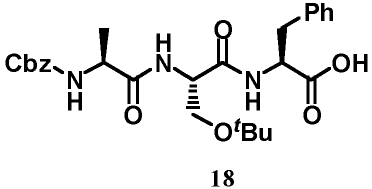

Cbz-Ala-Ser(tBu)-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Phe-OH (1 mmol), tributylamine (1 mmol), and 5 mL of dichloromethane were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 95%.

The experimental data of magnetic resonance imaging and mass spectrometry of the product were as follows:
**¹H NMR** (400 MHz, DMSO-*d*₆) δ 7.97 (d, *J =* 7.8 Hz, 1H), 7.74 (d, *J =* 7.7 Hz, 1H), 7.49 (d, *J* = 7.3 Hz, 1H), 7.39 - 7.14 (m, 10H), 5.02 (s, 2H), 4.53 - 4.44 (m, 1H), 4.38 - 4.30 (m, 1H), 4.16 - 4.07 (m, 1H), 3.49 - 3.38 (m, 2H), 3.10 - 3.03 (m, 1H), 2.97 - 2.88 (m, 1H), 1.19 (d, *J =* 6.9 Hz, 3H), 1.09 (s, 9H).
**¹³C NMR** (101 MHz, DMSO) δ = 172.4, 172.3, 169.5, 155.7, 137.3, 137.0, 129.2, 128.3, 128.2, 127.8, 127.7, 126.4, 72.9, 65.4, 61.7, 53.3, 53.0, 50.1, 36.9, 27.1, 18.1.
**HRMS (ESI)** m/z calcd. for C₂₇H₃₅N₃NaO₇ [M+Na]⁺: 536.2367, found: 536.2372.

### EXAMPLE 19

In this example, a polypeptide of Formula 19 was prepared, and the specific process was as follows:

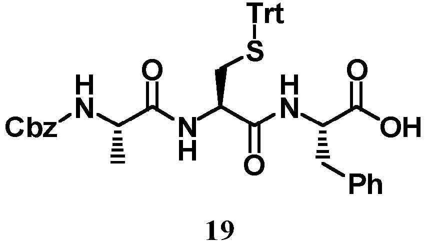

Cbz-Ala-Cys(Trt)-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Phe-OH (1 mmol), N-methylmorpholine (0.5 mmol), triethylamine (1 mmol), and 5 mL of dichloromethane were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 95%.

The experimental data of magnetic resonance imaging and mass spectrometry of the product were as follows:
**¹H NMR** (400 MHz, DMSO-*d*₆) δ 8.18 - 7.99 (m, 2H), 7.56 (d, *J =* 7.4 Hz, 1H), 7.36 - 7.14 (m, 25H), 5.12 - 4.91 (m, 2H), 4.46 - 4.32 (m, 2H), 4.14 - 4.04 (m, 1H), 3.09 - 2.99 (m, 1H), 2.95 - 2.85 (m, 1H), 2.44 - 2.30 (m, 2H), 1.19 (d, *J =* 7.2 Hz, 3H).
**¹³C NMR** (101 MHz, DMSO) δ = 172.4, 172.3, 169.5, 155.8, 144.3, 137.2, 137.0, 129.1, 129.1, 128.4, 128.2, 128.1, 127.8, 127.8, 126.8, 126.5, 65.8, 65.5, 53.5, 51.6, 50.2, 36.7, 33.8, 18.2.
**HRMS (ESI)** m/z calcd. for C₄₂H₄₁N₃NaO₆S [M+Na]⁺: 738.2608, found: 738.2615.

### EXAMPLE 20

In this example, a polypeptide of Formula 20 was prepared, and the specific process was as follows:

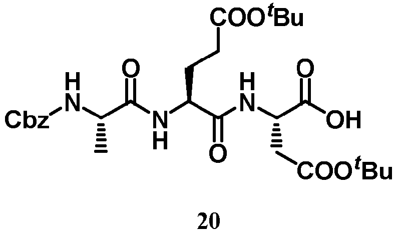

Cbz-Ala-Glu(tBu)-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Asp(tBu)-OH (1 mmol), N,N-dimethylaniline (0.5 mmol), triethylamine (1 mmol), and 5 mL of dichloromethane were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 97%.

The experimental data of magnetic resonance imaging and mass spectrometry of the product were as follows:
**¹H NMR** (400 MHz, DMSO-*d*₆) δ 8.19 (d, *J =* 7.9 Hz, 1H), 7.98 (d, *J =* 8.1 Hz, 1H), 7.47 (d, *J* = 7.5 Hz, 1H), 7.39 - 7.28 (m, 5H), 5.07 - 4.99 (m, 2H), 4.60 - 4.52 (m, 1H), 4.37 - 4.28 (m, 1H), 4.14 - 4.05 (m, 1H), 2.74 - 2.66 (m, 1H), 2.61 - 2.52 (m, 1H), 2.31 - 2.16 (m, 2H), 1.98 - 1.83 (m, 1H), 1.81 - 1.63 (m, 1H), 1.42 - 1.34 (m, 18H), 1.21 (d, *J =* 7.2 Hz, 3H).
**¹³C NMR** (101 MHz, DMSO) δ = 172.4, 172.1, 171.8, 170.8, 169.2, 155.7, 137.0, 128.4, 127.8, 127.8, 80.4, 79.6, 65.4, 51.4, 50.1, 48.7, 37.2, 31.1, 27.8, 27.8, 27.7, 18.1.
**HRMS (ESI)** m/z calcd. for C₂₈H₄₁N₃NaO₁₀ [M+Na]⁺: 602.2684, found: 602.2690.

### EXAMPLE 21

In this example, a polypeptide of Formula 21 was prepared, and the specific process was as follows:

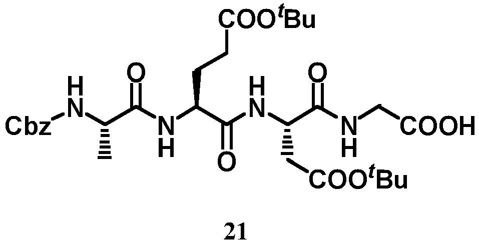

Cbz-Ala-Glu(tBu)-Asp(tBu)-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Gly-OH (1 mmol), 2,6-dimethylpyridine (2 mmol), and 5 mL of dichloromethane were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 93%.

The experimental data of magnetic resonance imaging and mass spectrometry of the product were as follows:
**¹H NMR** (400 MHz, DMSO-*d*₆) δ 8.14 (d, *J =* 8.0 Hz, 1H), 8.10 - 7.95 (m, 2H), 7.47 (d, *J =* 7.2 Hz, 1H), 7.40 - 7.22 (m, 5H), 5.10 - 4.94 (m, 2H), 4.67 - 4.55 (m, 1H), 4.30 - 4.21 (m, 1H), 4.13 - 4.04 (m, 1H), 3.82 - 3.67 (m, 2H), 2.76 - 2.62 (m, 1H), 2.50 - 2.36 (m, 1H), 2.32 - 2.10 (m, 2H), 2.02 - 1.82 (m, 1H), 1.82 - 1.62 (m, 1H), 1.39 (s, 9H), 1.37 (s, 9H), 1.20 (d, *J =* 6.9 Hz, 3H).
**¹³C NMR** (101 MHz, DMSO) δ = 172.8, 171.8, 170.9, 170.8, 170.4, 169.2, 155.8, 137.0, 128.3, 127.8, 127.8, 80.2, 79.6, 65.5, 52.0, 50.1, 49.4, 40.9, 37.4, 31.1, 27.8, 27.7, 27.3, 18.0.
**HRMS (ESI)** m/z calcd. for C₃₀H₄₄N₄NaO₁₁ [M+Na]⁺: 659.2899, found: 659.2905.

### EXAMPLE 22

In this example, a polypeptide of Formula 22 was prepared, and the specific process was as follows:

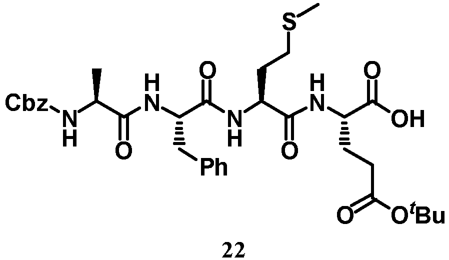

Cbz-Ala-Phe-Met-OH (0.5 mmol) and N-methyl-N-p-toluenesulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the product was transferred to a system, in which H-Glu(tBu)-OH (1 mmol), pyridine (2 mmol), and 5 mL of dichloromethane were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, *dr >* 99 : 1, with a yield of 92%.

The experimental data of magnetic resonance imaging and mass spectrometry of the product were as follows:
**¹H NMR** (400 MHz, DMSO-*d*₆) δ 8.13 (d, *J =* 7.5 Hz, 1H), 8.07 (d, *J =* 7.4 Hz, 1H), 7.93 (d, *J* = 7.3 Hz, 1H), 7.43 (d, *J =* 7.1 Hz, 1H), 7.40 - 7.28 (m, 5H), 7.25 - 7.15 (m, 5H), 5.09 - 4.93 (m, 2H), 4.60 - 4.49 (m, 1H), 4.44 - 4.36 (m, 1H), 4.28 - 4.20 (m, 1H), 4.05 - 3.98 (m, 1H), 3.09 - 3.00 (m, 1H), 2.90 - 2.80 (m, 1H), 2.49 - 2.40 (m, 2H), 2.33 - 2.23 (m, 2H), 2.03 (s, 3H), 2.02 - 1.89 (m, 2H), 1.88 - 1.74 (m, 2H), 1.39 (s, 9H), 1.14 (d, *J =* 7.0 Hz, 3H).
**¹³C NMR** (101 MHz, DMSO) δ = 173.0, 172.4, 171.5, 170.9, 170.7, 155.7, 137.6, 136.9, 129.3, 128.3, 127.9, 127.8, 127.7, 126.2, 79.8, 65.5, 53.6, 51.7, 51.1, 50.3, 37.2, 32.2, 31.2, 29.3, 27.7, 26.3, 18.0, 14.6.
**HRMS (ESI)** m/z calcd. for C₃₄H₄₆N₄NaO₉S [M+Na]⁺: 709.2878, found: 709.2884.

### EXAMPLE 23

In this example, an amide of Formula 23 was prepared, and the specific process was as follows:

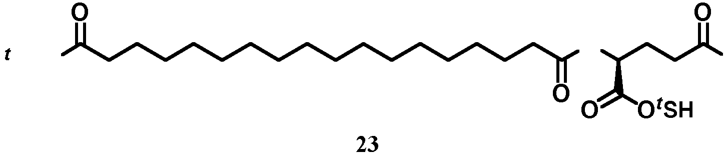

18-(Tert-butoxy)-18-oxooctadecanoic acid (0.5 mmol) and N-methyl-N-methylsulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the solvent was removed by distillation under reduced pressure, and the remaining mixture was transferred to a system, in which H-Glu-OtBu (1 mmol), N,N-diisopropylethylamine (1 mmol), 3 mL of dimethylsulfoxide, and 3 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, with a yield of 95%.

The experimental data of magnetic resonance imaging and mass spectrometry of the product were as follows:
**¹H NMR** (400 MHz, DMSO-*d*₆) δ 8.00 (d, *J =* 7.6 Hz, 1H), 4.16 - 4.09 (m, 1H), 2.29 - 2.23 (m, 2H), 2.12 (dt, *J =* 21.7, 7.3 Hz, 4H), 1.90 (dt, *J =* 13.5, 6.6 Hz, 1H), 1.75 (dd, *J =* 14.1, 6.7 Hz, 1H), 1.52 - 1.44 (m, 4H), 1.41 - 1.37 (m, 18H), 1.27 - 1.21 (m, 24H).
**¹³C NMR** (101 MHz, DMSO) δ 173.53, 172.25, 172.08, 171.03, 80.26, 79.16, 79.14, 51.81, 34.97, 34.74, 29.96, 29.00, 28.96, 28.91, 28.82, 28.76, 28.60, 28.53, 28.33, 27.68, 27.55, 26.24, 25.20, 24.55.
**HRMS (ESI)** m/z calcd. for C₃₁H₅₇NNaO₇ [M+Na]⁺: 578.4027, found: 578.4034.

### EXAMPLE 24

In this example, an amide of Formula 24 was prepared, and the specific process was as follows:

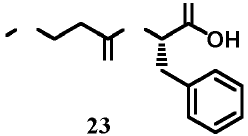

Fmoc-β-Ala-OH (0.5 mmol) and N-methyl-N-methylsulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the solvent was removed by distillation under reduced pressure, and the remaining mixture was transferred to a system, in which H-Phe-OH (1 mmol), N,N-diisopropylethylamine (1 mmol), 3 mL of acetonitrile, and 3 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, with a yield of 90%.

The experimental data of magnetic resonance imaging and mass spectrometry of the product were as follows:
**¹H NMR** (400 MHz, DMSO-*d*₆) δ 8.24 (d, *J =* 7.6 Hz, 1H), 7.88 (d, *J =* 7.5 Hz, 2H), 7.69 (d, *J* = 7.2 Hz, 2H), 7.41 (t, *J =* 7.4 Hz, 2H), 7.33 (t, *J =* 7.4 Hz, 2H), 7.29 - 7.13 (m, 6H), 4.53 - 4.42 (m, 1H), 4.29 (d, *J =* 6.5 Hz, 2H), 4.22 (d, *J =* 6.4 Hz, 1H), 3.21 - 3.05 (m, 3H), 2.89 (dd, *J =* 13.2, 9.4 Hz, 1H), 2.30 (dq, *J =* 19.6, 13.0, 10.2 Hz, 2H).
**¹³C NMR** (101 MHz, DMSO) δ 173.05, 170.26, 155.99, 143.90, 140.72, 137.67, 129.06, 128.12, 127.59, 127.05, 126.36, 125.15, 120.07, 65.39, 53.43, 46.75, 36.98, 36.84, 35.40.
**HRMS (ESI)** m/z calcd. for C₂₇H₂₆N₂NaO₅ [M+Na]⁺: 481.1734, found: 481.1739.

### EXAMPLE 25

In this example, an amide of Formula 25 was prepared, and the specific process was as follows:

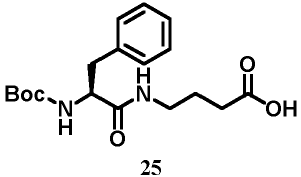

Boc-Phe-OH (0.5 mmol) and N-methyl-N-methylsulfonyl ethynylamine (0.55 mmol) were taken and dissolved in 5 mL of dichloromethane, and the mixture was reacted under stirring at room temperature. The reaction process was monitored by TLC. When the raw material acid was completely consumed, the solvent was removed by distillation under reduced pressure, and the remaining mixture was transferred to a system, in which γ-aminobutyric acid (1 mmol), triethylamine (1 mmol), and 3 mL of N,N-dimethylformamide, and 3 mL of water were mixed under stirring in advance, and reacted. The reaction process was monitored by TLC. After the reaction was completed, 15 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product as a white solid, with a yield of 96%.

The experimental data of magnetic resonance imaging and mass spectrometry of the product were as follows:
**¹H NMR** (400 MHz, DMSO-*d*₆) δ 7.96 - 7.78 (m, 1H), 7.23 (td, *J =* 17.3, 15.8, 6.9 Hz, 5H), 6.81 (d, *J =* 8.3 Hz, 1H), 4.16 - 3.99 (m, 1H), 3.07 (dq, *J =* 11.8, 6.2 Hz, 2H), 2.92 (dd, *J =* 13.6, 4.8 Hz, 1H), 2.81 - 2.67 (m, 1H), 2.17 (t, *J =* 7.4 Hz, 2H), 1.59 (p, *J =* 6.6 Hz, 2H), 1.31 (s, 9H).
**¹³C NMR** (101 MHz, DMSO) δ 174.14, 171.41, 155.09, 138.08, 129.12, 127.94, 126.11, 77.95, 55.76, 37.88, 37.70, 30.91, 28.10, 24.47.
**HRMS (ESI)** m/z calcd. for C₁₈H₂₆N₂NaO₅ [M+Na]⁺: 373.1734, found: 373.1738.

### EXAMPLE 26

In this example, carfilzomib was prepared, and the specific process was as follows:

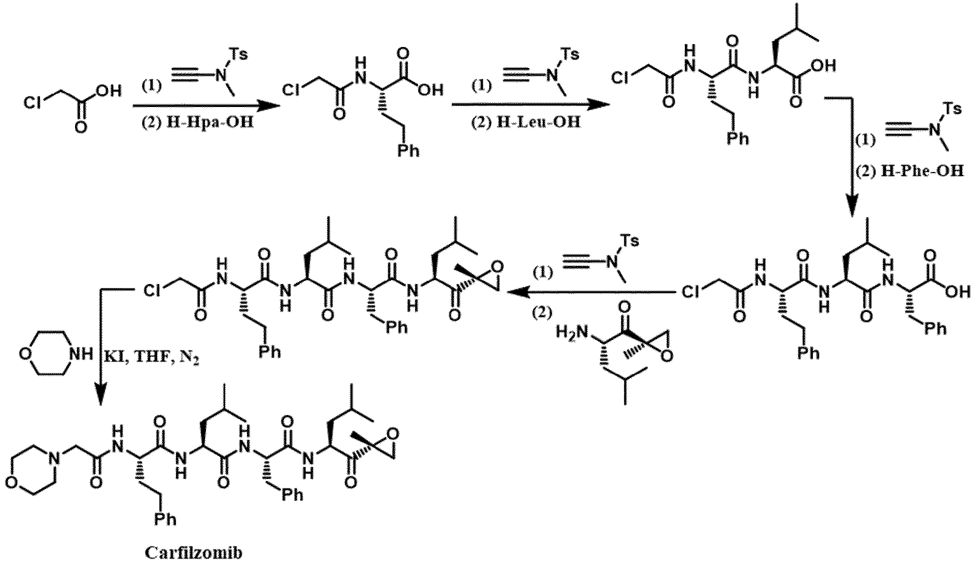

Chloroacetic acid (5 mmol) and N-methyl-N-p-toluenesulfonyl ethynylamine (5.5 mmol) were taken and dissolved in 5 mL of dichloromethane, and the mixture was reacted under stirring at room temperature. The reaction process was monitored by TLC. When the raw material acid was completely consumed, the product was transferred to a system, in which L-homophenylalanine (10 mmol), triethylamine (10 mmol), acetonitrile (10 mL), and water (5 mL) were mixed under stirring in advance, and reacted. The reaction process was monitored by TLC. After the reaction was completed, 20 mL of water was added, the product was acidified to pH = 2 with 1 M hydrochloric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining a pure product. The above steps were repeated for condensation with L-phenylalanine, L-leucine, and (2S)-2-amino-4-methyl-1-[(2R)-2-methyloxiranyl]-1-pentanone trifluoroacetate in order to finally obtain an intermediate, which was reacted with morpholine (10 mmol), potassium iodide (KI, 5 mmol) and tetrahydrofuran (THF, 30 mL) in a reaction flask under stirring at room temperature under nitrogen protection. The reaction process was monitored by TLC and HPLC. After the reaction was completed, the reaction liquid was concentrated, water and ethyl acetate were added, the mixture was extracted three times with ethyl acetate, the organic phases were combined and dried over anhydrous magnesium sulfate, and the reaction liquid was concentrated and recrystallized with ethyl acetate and petroleum ether to obtain carfilzomib as a white solid with an overall yield of 70%. The structural formula and the experimental data of magnetic resonance imaging and mass spectrometry of the product were as follows:
**¹H NMR** (400 MHz, DMSO-*d*₆) δ 8.25 (d, *J =* 6.8 Hz, 1H), 8.09 (d, *J =* 7.7 Hz, 1H), 7.97 (d, *J* = 7.7 Hz, 1H), 7.90 (d, *J =* 7.6 Hz, 1H), 7.33 - 7.23 (m, 2H), 7.23 - 7.02 (m, 8H), 4.61 - 4.49 (m, 1H), 4.43 - 4.32 (m, 2H), 4.32 - 4.23 (m, 1H), 3.71 - 3.51 (m, 4H), 3.41 - 3.32 (m, 1H), 3.12 (d, *J =* 4.2 Hz, 1H), 3.06 - 2.85 (m, 4H), 2.81 - 2.70 (m, 1H), 2.57 - 2.51 (m, 1H), 2.47 - 2.38 (m, 4H), 1.93 - 1.76 (m, 2H), 1.69 - 1.59 (m, 1H), 1.55 - 1.47 (m, 1H), 1.43 - 1.28 (m, 7H), 0.89 - 0.83 (m, 6H), 0.83 - 0.76 (m, 6H).
**¹³C NMR** (100 MHz, DMSO-*d*₆) δ 208.2, 171.5, 171.0, 170.8, 168.8, 141.4, 137.3, 129.1, 128.2, 128.2, 127.8, 126.1, 125.8, 66.1, 61.3, 58.7, 53.2, 53.0, 51.8, 51.5, 51.1, 49.2, 40.7, 38.5, 37.4, 34.3, 31.4, 24.5, 24.1, 23.2, 23.0, 21.6, 21.0, 16.3.
**HRMS** (ESI) m/z calcd. for C₄₀H₅₈N₅O₇ [M+H]⁺: 720.4331, found: 720.4335.

### EXAMPLE 27

In this example, octreotide was prepared, and the specific process was as follows:

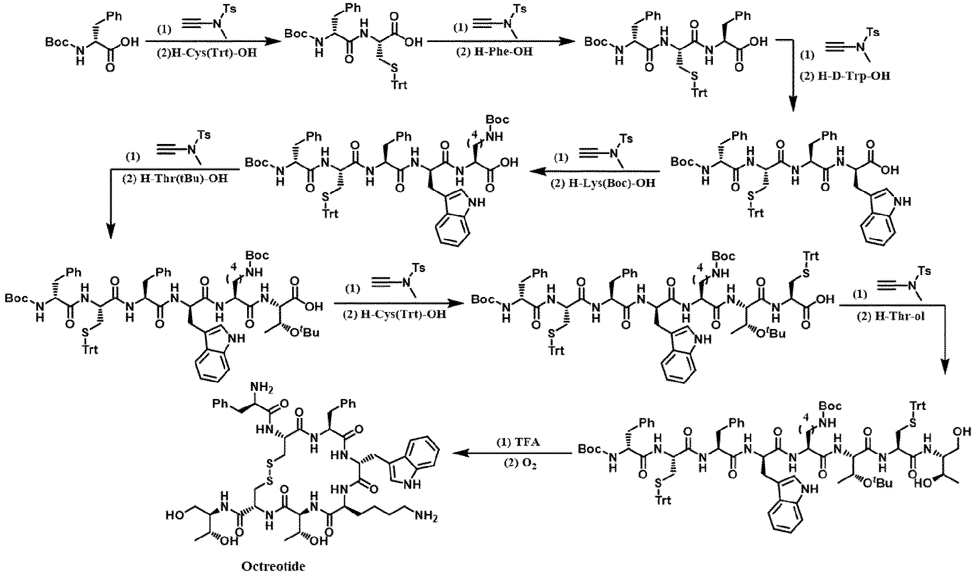

Boc-D-Phe-OH (5 mmol) and N-methyl-N-p-toluenesulfonyl ethynylamine (5.5 mmol) were taken and dissolved in 20 mL of dichloromethane, and the mixture was reacted under stirring at room temperature. The reaction process was monitored by TLC. When the raw material acid was completely consumed, the solvent was removed by distillation under reduced pressure, and the product was transferred to a system, in which H-Cys(Trt)-OH (10 mmol), N,N-diisopropylethylamine (10 mmol), acetonitrile (40 mL), and water (20 mL) were mixed under stirring in advance, and reacted. The reaction process was monitored by TLC. After the reaction was completed, 50 mL of water was added, the product was acidified to pH = 3 with 10% citric acid, the aqueous phase was extracted twice with ethyl acetate, the organic phases were combined, washed once with water and once with a saturated table salt solution, dried over anhydrous sodium sulfate, distilled under reduced pressure to remove the solvent, recrystallized with dichloromethane and diethyl ether, and filtered to collect a solid, thus obtaining Boc-D-Phe-Cys(Trt)-OH. The above steps were repeated for condensation with H-D-Trp-OH, H-Lys(Boc)-OH, H-Thr(tBu)-OH, H-Cys(Trt)-OH, and L-threoninol in order to obtain a protected linear chain Boc-D-Phe-Cys(Trt)-Phe-D-Trp-Lys(Boc)-Thr(tBu)-Cys(Trt)-Thr-ol of octreotide, and the intermediate was added to a reaction flask, in which trifluoroacetic acid (42.5 mL), 1,2-ethanedithiol (2.5 mL), triisopropylsilane (2.5 mL) and water (2.5 mL) had been mixed in advance, and stirred. The reaction was monitored by TLC and HPLC. After the reaction was completed, recrystallization with diethyl ether was carried out to obtain a linear chain of octreotide. Finally, the linear chain of octreotide was dissolved in water and diluted to a concentration of 10⁻⁴ M, the solution was adjusted to pH = 8 with 1 mmol aqueous ammonia, air was introduced for oxidation for 48 h at room temperature, and after freeze-drying, octreotide was obtained as a white solid with an overall yield of 45%.

The structural formula and the experimental data of magnetic resonance imaging and mass spectrometry of the product were as follows:
**¹H NMR** (400 MHz, DMSO-*d*₆) δ 10.88 (s, 1H), 9.04 (d, *J =* 8.2 Hz, 1H), 8.70 (d, *J =* 4.5 Hz, 1H), 8.52 (d, *J =* 7.5 Hz, 1H), 8.40 (d, *J =* 7.3 Hz, 1H), 8.34 (d, *J =* 8.4 Hz, 1H), 8.28 - 8.09 (m, 3H), 7.98 - 7.85 (m, 3H), 7.71 (d, *J =* 8.5 Hz, 1H), 7.45 (d, *J =* 7.8 Hz, 1H), 7.39 - 7.26 (m, 8H), 7.21 - 7.18 (m, 2H), 7.14 - 7.05 (m, 4H), 7.02 - 6.97 (m, 2H), 5.09 (d, *J =* 4.0 Hz, 1H), 5.03 - 4.97 (m, 1H), 4.90 - 4.85 (m, 1H), 4.83 - 4.72 (m, 1H), 4.64 (s, 1H), 4.42 (dd, *J =* 8.4, 5.4 Hz, 1H), 4.24 (dd, *J =* 14.5, 7.5 Hz, 2H), 4.12 - 4.06 (m, 1H), 4.00 - 3.92 (m, 2H), 3.66 - 3.61 (m, 1H), 3.18 (dd, *J =* 13.8, 5.2 Hz, 1H), 3.02 - 2.95 (m, 3H), 2.92 - 2.72 (m, 7H), 2.65 - 2.57 (m, 2H), 1.73 - 1.64 (m, 1H), 1.42 - 1.31 (m, 3H), 1.11 (d, *J =* 6.2 Hz, 3H), 1.05 (d, *J =* 6.4 Hz, 3H), 0.89 - 0.77 (m, 2H).
**¹³C NMR** (101 MHz, DMSO-*d*₆) δ 172.53, 171.87, 170.65, 170.39, 169.59, 168.47, 168.10, 136.81, 136.14, 134.76, 129.66, 129.04, 128.54, 128.09, 127.23, 127.05, 126.44, 123.69, 120.90, 118.26, 118.18, 111.34, 109.03, 66.63, 64.23, 60.20, 58.58, 55.90, 55.22, 53.97, 53.36, 53.23, 52.55, 52.01, 44.17, 42.39, 38.78, 38.52, 37.42, 30.26, 26.44, 26.38, 22.03, 19.92, 19.50.
**HRMS** (ESI) m/z calcd. for C₄₉H₆₇N₁₀O₁₀S₂[M+H]⁺: 1019.4478, found: 1019.4483.

The present invention provides a method for synthesizing an amide and/or a polypeptide using an unprotected amino acid as an ammonia component. In the preparation method for the polypeptide, after a peptide bond is constructed, the next amino acid can be directly condensed without deprotection operation, thereby reducing many unnecessary protecting agents, deprotecting agents and purification solvents, and reducing the use of chemicals and the generation of chemical wastes. Furthermore, the cost of polypeptide synthesis is greatly reduced, the atomic economy and step economy of polypeptide synthesis are improved, the efficiency of polypeptide synthesis is greatly improved, energy conservation and emission reduction are efficiently achieved, so that it has broad industrial application prospects.

The examples of the present invention have been described in detail above; however, the present invention is not limited to the above examples, and various changes can also be made within the knowledge of those of ordinary skill in the related technical field without departing from the gist of the present invention. In addition, the examples of the present invention and the features in the examples can be combined with each other without conflict.

## Claims

1. A method for preparing an amide and/or a polypeptide, comprising the following steps: 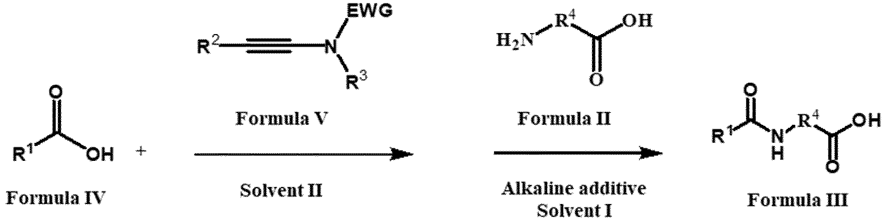
reacting a compound of Formula IV with a compound of Formula V in solvent II, and then carrying out a nucleophilic substitution reaction with a compound of Formula II and an alkaline additive in solvent I to prepare a compound of Formula III;
wherein R¹ is selected from alkyl, alkenyl, alkynyl, cycloalkyl, heterocycloalkyl, aryl, heteroaryl, an amino acid residual body lacking C-terminal carboxyl, an amino acid derivative residual body or a polypeptide fragment lacking C-terminal carboxyl; R² is selected from hydrogen, deuterium, alkyl, cycloalkyl, heterocycloalkyl, aryl, heteroaryl, silyl, or alkylsilyl; R³ is selected from alkyl, cycloalkyl, heterocycloalkyl, aryl, or heteroaryl; R⁴ is selected from alkyl, cycloalkyl, heterocycloalkyl, aryl, heteroaryl, an amino acid residue, or a polypeptide fragment; and EWG is selected from nitro, cyano, sulfonyl, arylsulfonyl, alkanoyl, and phosphonyl; wherein the alkyl, alkenyl, alkynyl, cycloalkyl, heterocycloalkyl, aryl, heteroaryl, silyl, and alkylsilyl are optionally unsubstituted or substituted with one or more R¹¹, with each R¹¹ being independently selected from halogen, hydroxyl, carbonyl, and C₁-C₆ alkyl.

2. The method for preparing an amide and/or a polypeptide according to claim 1, wherein when the solvent II is different from the solvent I, the method for preparing the amide and/or polypeptide further comprises after the reaction, removing the solvent II before the nucleophilic substitution reaction.

3. A method for preparing an amide and/or a polypeptide, comprising the following steps: 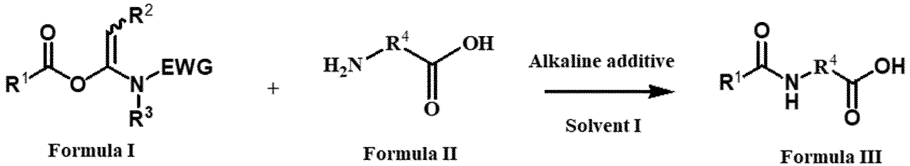
dissolving a compound of Formula I, a compound of Formula II and an alkaline additive in solvent I for a nucleophilic substitution reaction to prepare a compound of Formula III,
wherein R¹ is selected from alkyl, alkenyl, alkynyl, cycloalkyl, heterocycloalkyl, aryl, heteroaryl, an amino acid residual body lacking C-terminal carboxyl, an amino acid derivative residual body or a polypeptide fragment lacking C-terminal carboxyl; R² is selected from hydrogen, deuterium, alkyl, cycloalkyl, heterocycloalkyl, aryl, heteroaryl, silyl, or alkylsilyl; R³ is selected from alkyl, cycloalkyl, heterocycloalkyl, aryl, or heteroaryl; R⁴ is selected from alkyl, cycloalkyl, heterocycloalkyl, aryl, heteroaryl, an amino acid residue, or a polypeptide fragment; and EWG is selected from nitro, cyano, sulfonyl, arylsulfonyl, alkanoyl, and phosphonyl; wherein the alkyl, alkenyl, alkynyl, cycloalkyl, heterocycloalkyl, aryl, heteroaryl, silyl, and alkylsilyl are optionally unsubstituted or substituted with one or more R¹¹, with each R¹¹ being independently selected from halogen, hydroxyl, carbonyl, and C₁-C₆ alkyl.

4. The method for preparing an amide and/or a polypeptide according to claim 1 or 3, wherein the alkaline additive includes an organic base and/or an inorganic base.

5. The method for preparing an amide and/or a polypeptide according to claim 4, wherein the organic base includes at least one of trimethylamine, triethylamine, tripropylamine, N,N-dimethylethylamine, N,N-diethylmethylamine, N,N-diisopropylethylamine, N-methylpyrrolidine, N-methylpiperidine, N-methylmorpholine, tributylamine, tribenzylamine, dimethylbenzylamine, N,N-dimethylaniline, pyridine, 2-methylpyridine, 2,6-dimethylpyridine, 4-dimethylaminopyridine, quinoline, quinine, tetramethylguanidine, imidazole, 1,8-diazabicyclo[5.4.0]undec-7-ene, triethylene diamine, tetramethylethylenediamine, tetraethylethylenediamine, tetramethylpropylenediamine, tetraethylpropylenediamine, tetrabutylammonium iodide, tetrabutylammonium fluoride, tetrabutylammonium chloride, tetrabutylammonium bromide, or tetrabutylammonium hydroxide; and the inorganic base includes at least one of sodium bicarbonate, sodium carbonate, lithium bicarbonate, lithium carbonate, potassium bicarbonate, potassium carbonate, cesium bicarbonate, cesium carbonate, sodium hydroxide, potassium hydroxide, lithium hydroxide, calcium bicarbonate, or calcium hydroxide.

6. The method for preparing an amide and/or a polypeptide according to claim 1 or 3, wherein the molar ratio of the alkaline additive to the compound of Formula II is (0.1-20) : 1.

7. The method for preparing an amide and/or a polypeptide according to claim 1 or 3, wherein the solvent I includes at least one of water, acetonitrile, ethanol, isopropanol, tert-butanol, ethylene glycol, 1,2-propanediol, 2,3-butanediol, diethyl ether, ethylene glycol dimethyl ether, ethylene glycol diethyl ether, dioxane, dimethyl sulfoxide, N,N-dimethylformamide, N,N-dimethylacetamide, N-methylpyrrolidone, toluene, m-xylene, dichloromethane, 1,2-dichloroethane, or chloroform; and the solvent II includes at least one of dichloromethane, chloroform, 1,2-dichloroethane, diethyl ether, toluene, acetonitrile, methanol, or ethanol.

8. The method for preparing an amide and/or a polypeptide according to claim 1 or 3, wherein the nucleophilic substitution reaction is carried out at a temperature of -20°C to 150°C.

9. The method for preparing an amide and/or a polypeptide according to claim 1 or 3, wherein the compound of Formula II includes any one of an amino acid compound, a polypeptide compound with both free amino and carboxyl groups, or other compounds with both amino and carboxyl groups.

10. The method for preparing an amide and/or a polypeptide according to claim 3, wherein the method for preparing the amide and/or the polypeptide comprises the following steps: dissolving a compound of Formula II and an alkaline additive in solvent I, and then adding a compound of Formula I for a nucleophilic substitution reaction to prepare a compound of Formula III.

11. The method for preparing an amide and/or a polypeptide according to claim 3, wherein a preparation method for the compound of Formula I comprises the following steps: 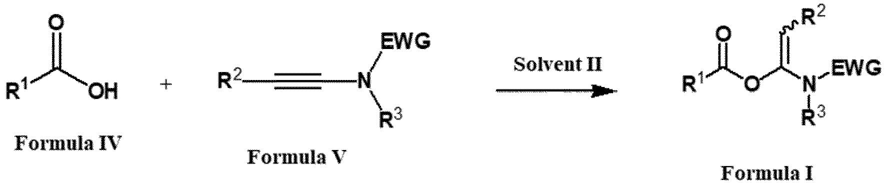
dissolving a compound of Formula IV and a compound of Formula V in solvent II to obtain a mixture, and reacting the mixture under stirring to prepare the compound of Formula I;
wherein R¹, R², R³ and EWG are defined as in claim 3.

12. The method for preparing an amide and/or a polypeptide according to claim 1 or 3, wherein the method for preparing the amide and/or the polypeptide comprises the following steps: 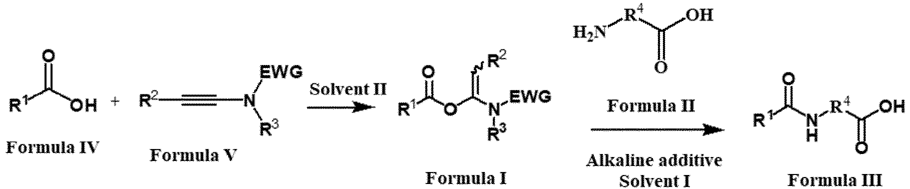
S1: dissolving a compound of Formula IV and a compound of Formula V in solvent II to obtain a mixture, and reacting the mixture under stirring to prepare a compound of Formula I; and
S2: dissolving the compound of Formula I, a compound of Formula II and an alkaline additive in solvent I for a nucleophilic substitution reaction to prepare a compound of Formula III;
wherein R¹, R², R³, R⁴ and EWG are defined as in claim 1 or 3.

13. The method for preparing an amide and/or a polypeptide according to claim 12, wherein the compound of Formula IV includes at least one of a fatty acid, an aromatic acid, a heterocyclic acid, an acetylenic acid, an alkenoic acid, an amino acid, a polypeptide carboxylic acid, and derivatives of the above compounds.

14. The method for preparing an amide and/or a polypeptide according to claim 12, wherein the molar ratio of the compound of Formula IV to the compound of Formula V is 1 : (0.1-5).

15. Use of the method for preparing an amide and/or a polypeptide according to any one of claims 1-14 in the preparation of an amide and/or a polypeptide.
